(19) **Europäisches Patentamt**
**European Patent Office**
**Office européen des brevets**

(11) **EP 2 222 627 B3**

(12) **NOUVEAU FASCICULE DE BREVET EUROPEEN**
Après la procédure de limitation (B3-1)

(45) Mention de la délivrance du brevet:
**08.08.2012 Bulletin 2012/32**

(45) Date de publication et mention de la décision de limitation:
**B3-1 08.04.2015 Bulletin 2015/15**

(21) Numéro de dépôt: **08861391.4**

(22) Date de dépôt: **09.12.2008**

(51) Int Cl.:
*C07C 51/367* (2006.01)    *C07C 59/52* (2006.01)
*C07C 59/64* (2006.01)    *C07C 45/29* (2006.01)
*C07C 47/575* (2006.01)    *C07C 47/58* (2006.01)

(86) Numéro de dépôt international:
**PCT/EP2008/067107**

(87) Numéro de publication internationale:
**WO 2009/077383 (25.06.2009 Gazette 2009/26)**

(54) **PROCEDE DE PREPARATION DE COMPOSES P-HYDROXYMANDELIQUES EVENTUELLEMENT SUBSTITUES ET DERIVES**

VERFAHREN ZUR HERSTELLUNG VON OPTIONAL SUBSTITUIERTEN P-HYDROXYMANDELSÄUREVERBINDUNGEN UND DERIVATEN DARAUS

METHOD FOR PREPARING OPTIONALLY SUBSTITUTED P-HYDROXYMANDELIC COMPOUNDS AND DERIVATIVES THEREOF

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **18.12.2007 FR 0708823**

(43) Date de publication de la demande:
**01.09.2010 Bulletin 2010/35**

(73) Titulaire: **Rhodia Opérations**
**93306 Aubervilliers (FR)**

(72) Inventeurs:
• **HEINISCH, Bruno**
  **F-69100 Lyon (FR)**
• **PITIOT, Pascal**
  **F-69008 Lyon (FR)**
• **GRIENEISEN, Jean-Louis**
  **F-69960 Corbas (FR)**

(74) Mandataire: **Menville, Laure et al**
**Rhodia Opérations**
**IAM**
**85, avenue des frères Perret**
**69192 Saint-Fons (FR)**

(56) Documents cités:
**FR-A- 2 379 501**

Remarques:
Le dossier contient des informations techniques présentées postérieurement au dépôt de la demande et ne figurant pas dans le présent fascicule.

**Description**

La présente invention a pour objet un procédé de préparation de vanilline ou d'éthylvaniline. Dans l'exposé qui suit de l'invention, on entend par "composés p-hydroxymandéliques éventuellement substitués" un composé aromatique au moins porteur d'un groupe -CHOH-COOH en position para d'un groupe hydroxyle.

La vanilline est obtenue à partir de sources naturelles telles que la lignine, l'acide férulique mais une part importante de la vanilline est produite par voie chimique.

De nombreuses méthodes de préparation diverses et variées, sont décrites dans la littérature (KIRK-OTHMER - Encyclopedia of Chemical Technology 24, p 812-825, 4ème édition (1997)).

Une voie d'accès classique à la vanilline implique une réaction de condensation d'acide glyoxylique sur le gaïacol en milieu basique, pour obtenir l'acide 4-hydroxy-3-méthoxymandélique. Ce produit est ensuite oxydé pour conduire à la vanilline.

Le rendement de la condensation est limité par le fait que la réaction de condensation n'est pas sélective et conduit également à l'acide o-hydroxymandélique ainsi qu'à des acides dimandéliques.

La formation des acides dimandéliques résulte d'une réaction subséquente, àsavoirune deuxième condensation d'acide glyoxylique sur un acide mandélique.

On cherche à limiter ces réactions subséquentes afin d'obtenir une sélectivité optimale.

Les procédés continus sont le plus souvent conduits dans unecascade de plusieurs réacteurs de type parfaitement agités. Cependant, la multiplication des réacteurs induit des coûts tant au niveau de la réalisation qu'au niveau de l'exploitation.

L'invention a pour but de proposer un procédé de préparation de composés mandéliques permettant de surmonter un ou plusieurs des inconvénients évoqués ci-dessus, et en particulier d'obtenir une sélectivité améliorée.

L'objet de la présente invention est donc un procédé de préparation de vaniline ou l'éthylvaniline, comprenant la condensation dans l'eau, en présence d'un agent alcalin, de gaïacol ou de guétol, avec l'acide glyoxylique, qui est caractérisé par le fait que la réaction est conduite dans un réacteur à écoulement piston, et l'oxydation de l'acide 4-hydroxy-3-méthoxymandélique ou de l'acide 3-éthoxy-4-hydroxymandélique obtenu.

Selon des modes de réalisation préférés, le réacteur est un réacteur tubulaire ou un réacteur colonne.

Dans l'exposé qui suit la présente invention, on entend par « réacteur tubulaire », un réacteur en forme de tube et par « réacteur colonne » un réacteur vertical de section circulaire.

Par « écoulement piston », on définit un écoulement unidirectionnel dans lequel dans un plan perpendiculaire à l'écoulement, tous les filets fluides se déplacent avec une vitesse uniforme et toutes les grandeurs physiques y sont identiques. Dans un tel écoulement, le mélange radial est parfait alors qu'il n'y a pas de mélange axial. En pratique, ces conditions sont considérées comme remplies lorsque l'écoulement est turbulent.

On estime qu'un écoulement est turbulent lorsque le nombre de Reynolds est supérieur ou égal à 2000, et préférentiellement lorsqu'il est supérieur à 5000.

On rappelle que la définition du nombre de Reynolds est :

$$Re = \frac{\rho \cdot v \cdot d}{\mu}$$

dans laquelle:

- $\rho$ est la masse volumique du fluide en kg/m$^3$ ;
- $v$ est la vitesse d'écoulement en m/s ;
- $d$ est le diamètre du réacteur en m
- $\mu$ est la viscosité dynamique en Pa.s

La figure 1 est une représentation schématique d'un réacteur tubulaire formé de tubes concentriques.
La figure 2 est une représentation schématique d'un réacteur en forme de colonne.
La figure 3 représente schématiquement un réacteur multi-tubulaire comprenant des tubes réunis en faisceau.

Conformément au procédé de l'invention, il a été trouvé que la mise en oeuvre du procédé décrit dans un réacteur tubulaire à écoulement piston permettait l'obtention d'une sélectivité améliorée. En effet, une bonne sélectivité a été obtenue en raison d'une limitation des réactions subséquentes.

Le procédé a par ailleurs comme avantage un faible encombrement ainsi qu'une économie de coûts de fonctionnement et d'investissement comparativement à une cascade de réacteurs parfaitement agités, équipés chacun de moyens d'introduction des réactifs, de soutirage des produits, ainsi que des dispositifs de mélange des réactifs et de contrôle

EP 2 222 627 B3

des paramètres de procédé.

Le procédé décrit propose de préparer des composés p-hydroxymandéliques en effectuant une réaction de condensation d'un composé aromatique porteur d'au moins un groupe hydroxyle, et de l'acide glyoxylique en présence d'un agent alcalin et éventuellement en présence d'un catalyseur.

Dans l'exposé qui suit, on entend par « composé aromatique » un composé cyclique présentant des doubles liaisons délocalisés tels que défini dans la littérature, notamment par M. SMITH et J. MARCH Advanced Organic Chemistry, 5ème Edition, John Wiley and Sons, 1992, pp.46 et suivantes.

Les composés auxquels s'applique le procédé de l'invention sont le gaïacol et le guétol. Conformément au procédé de l'invention, on effectue la condensation du composé aromatique hydroxylé et de l'acide glyoxylique, en phase liquide, en présence d'un agent alcalin.

Comme agents alcalins, on fait appel de préférence à un hydroxyde de métal alcalin qui peut être notamment l'hydroxyde de sodium ou de potassium. Pour des considérations économiques, on choisit préférentiellement l'hydroxyde de sodium. La présence de la base conduit à la salification du composé aromatique hydroxylé d'une part et de la fonction carboxylique d'autre part.

La solution d'hydroxyde de métal alcalin mise en oeuvre a une concentration généralement comprise entre 10 et 50 % en poids.

La quantité d'hydroxyde de métal alcalin introduite dans le milieu réactionnel tient compte de la quantité nécessaire pour salifier la fonction hydroxyle du composé aromatique hydroxylé et la fonction carboxylique de l'acide glyoxylique. Généralement, la quantité d'hydroxyde de métal alcalin varie entre 80 et 120 % de la quantité stoechiométrique.

L'acide glyoxylique peut être utilisé en solution aqueuse ayant une concentration variant par exemple, entre 15 et 70 % en poids. On a recours, d'une manière préférée, aux solutions commerciales dont la concentration est d'environ 50% en poids.

On fait réagir l'acide glyoxylique sur le composé aromatique hydroxylé. Le rapport molaire entre le composé aromatique hydroxylé et l'acide glyoxylique varie entre 1,0 et 4,0.

La concentration du composé aromatique hydroxylé est comprise de préférence entre 0,5 et 1,5 moles/litre, et plus particulièrement aux environs de 1 mole/litre.

Une variante possible consiste à effectuer la réaction en présence d'un catalyseur de type acide dicarboxylique, de préférence l'acide oxalique comme décrit dans WO 99/65853.

La quantité de catalyseur mise en oeuvre exprimée par le rapport entre le nombre de moles de catalyseur et le nombre de moles d'acide glyoxylique est choisie avantageusement entre 0,5 et 2,5 % de préférence, entre 1 et 2 %.

La température de la réaction est choisie avantageusement entre 20°C et 90°C, et de préférence entre 30°C et 40°C.

La réaction est conduite à pression atmosphérique mais sous atmosphère contrôlée de gaz inertes, de préférence d'azote ou éventuellement de gaz rares, en particulier l'argon. On choisit préférentiellement l'azote.

De préférence, le composé aromatique hydroxylé et la base sont mélangés au préalable. Cette variante permet de mieux maîtriser la température lors de la réaction exothermique de salification de l'acide glyoxylique.

Ainsi, selon un mode de réalisation préféré du procédé, le composé aromatique hydroxylé est d'abord mis en contact avec l'hydroxyde de métal alcalin en solution aqueuse.

La solution résultante est ensuite mise en contact avec l'acide glyoxylique et le mélange obtenu est ensuite introduit dans le réacteur à écoulement piston.

Le mélange obtenu a une viscosité à 20°C comprise entre 1,5 et 3 mPa.s.

Le débit d'introduction dudit mélange varie entre 0,010 à 20 m$^3$/h.

Le temps de séjour dans le réacteur est compris de préférence entre 10 min et 2 heures.

En fin de réaction, on sépare le composé p-hydroxymandélique obtenu du mélange réactionnel sous forme salifiée selon les techniques classiques de séparation, notamment par cristallisation ou par extraction à l'aide d'un solvant organique approprié.

Le procédé de l'invention conduit à l'obtention de l'acide 4-hydroxy-3-méthoxymandélique et de l'acide 3-éthoxy-4-hydroxymandélique.

Une application de l'invention est la préparation d'aldéhydes hydroxyaromatiques, paroxydation des composés p-hydroxymandéliques obtenus selon l'invention.

Le mélange réactionnel obtenu précédemment peut être utilisé tel quel. Toutefois, il est préférable de récupérer le composé aromatique hydroxylé qui n'a pas réagi.

A cet effet, on peut mettre en oeuvre les traitements décrits dans l'état de la technique notamment dans FR-A 2379501. Il consiste à ajouter un acide minéral, par exemple l'acide chlorhydrique ou sulfurique de manière à ajuster le pH entre 5 et 7 puis à extraire le composé aromatique hydroxylé qui n'a pas réagi dans un solvant organique, notamment éther, toluène...

Après extraction, les phases aqueuse et organique sont séparées.

La phase aqueuse comprend le composé p-hydroxymandélique qui va ensuite être oxydé

L'oxydation peut être conduite selon les techniques décrites dans la littérature. Ainsi, on peut se référer à P. HEBERT [Bull. Soc. Chim. France, 27, p.45-55(1920)] et à NAGAI SHIGEKI et al, [JP-A 76/128934]. L'oxydation est généralement conduite par l'oxygène ou l'air sous pression, en milieu basique et en présence d'un catalyseur approprié tel que par exemple, les dérivés du chrome, cobalt, cuivre, vanadium ou osmium.

Ainsi, l'invention permet d'accéder facilement à la vanilline et 3-éthyl-vanilline par oxydation respectivement des acides 4-hydroxy-3-méthoxymandélique et 3-éthoxy-4-hydroxy-mandélique.

Le réacteur dans lequel le procédé selon l'invention est mis en oeuvre est un réacteur à écoulement piston. Le plus souvent, le réacteur présentera un rapport longueur / diamètre supérieur à 3. Il peut s'agir notamment d'un réacteur tubulaire présentant un rapport longueur/ diamètre compris entre 4 et 30 et en particulier compris entre 5 et 10. Avantageusement, le réacteur tubulaire est formé de manière à avoir un faible encombrement, par exemple lorsqu'il est replié.

Le matériau du réacteur n'est pas particulièrement limité. Il sera choisi de manière à être inerte dans les conditions réactionnelles. Généralement, on choisira un réacteur en acier inoxydable.

Les réacteurs tubulaires sont généralement disposés à l'horizontale. Toutefois, afin de s'adapter aux contraintes spatiales, il peut également être envisagé de prévoir un réacteur disposé à la verticale ou encore incliné. De préférence, le réacteur est disposé de manière verticale.

Dans le cas d'un réacteur incliné ou vertical, il est préféré d'alimenter le réacteur par le dessous, afin de faciliter l'opération de dégazage.

Avantageusement, un ou plusieurs plateaux perforés sont disposés à proximité de l'entrée des réactifs afin d'assurer une bonne homogénéité des fluides dans cette section du réacteur.

Avantageusement, le réacteur tubulaire est en forme de colonne. Il est équipé de conduits d'entrée des réactifs et de sortie du mélange réactionnel. L'alimentation des réactifs est réalisée par des moyens classiques tels que par exemple une pompe et plus particulièrement une pompe à centrifuge ou une pompe volumétrique.

En principe, il est préféré de travailler en phase liquide seulement, en l'absence de phase gazeuse.

Le réacteur tubulaire peut être équipé d'un garnissage.

La présence dans le réacteur d'un garnissage créé des turbulences qui assurent une homogénéité du mélange réactionnel sur toute la section du réacteur. Le garnissage permet ainsi de maintenir le caractère d'écoulement piston, y compris pour des vitesses d'écoulement faibles, par exemple de l'ordre du mm/s.

Le matériau du garnissage importe peu, du moment qu'il est chimiquement inerte vis-à-vis du mélange réactionnel dans les conditions de la réaction. Généralement, il s'agit de matériaux tels que du verre, du métal, notamment de l'acier inoxydable, du carbone, du polymère ou encore de la céramique.

Différents types de garnissages peuvent être envisagés.

Il peut s'agir de garnissage en vrac, qui consiste en de petits objets par exemple sous forme d'anneaux, de selles, de boules ou cylindres creux, dont est rempli tout ou partie du réacteur.

De préférence, le garnissage est disposé dans le réacteur à proximité de l'entrée des réactifs.

Dans le cas d'un réacteur disposé à la verticale, le garnissage est disposé de préférence sur toute la hauteur du réacteur. Il y a alors lieu de prévoir un support approprié, par exemple sous forme de croisillons, afin de maintenir le garnissage en place.

Particulièrement préféré est un garnissage de type mélangeur statique, composé d'éléments de mélange comportant des lames de guidage arrangées selon des angles précis et disposées de manière complexe. Ce type de garnissage est par exemple commercialisé par la société SULZER sous la dénomination SMV et SMX.

Pour la description de ces garnissages, on peut se référer à l'article « Don't Be Baffled By Static Mixers » paru dans Chemical Engineering, May 2003.

D'un point de vue pratique, on choisit un tube linéaire sans garnissage replié sur lui-même et disposé horizontalement ou verticalement lorsque le nombre de Reynolds est supérieur à 2000, et préférentiellement lorsqu'il est supérieur à 5000. Lorsque le nombre de Reynolds est inférieur à 2000, on préfère mettre en oeuvre un réacteur avec garnissage.

Il est à noter qu'il est possible lorsque le réacteur est un tube replié sur lui-même de l'équiper partiellement d'un garnissage. Après chaque coude du tube, on introduit un tronçon de garnissage d'une longueur équivalente par exemple de 3 à 6 fois le diamètre du tube, sur toute ou partie de la longueur droite du tube située entre deux coudes successifs.

Les figures annexées illustrent le type d'appareillages susceptibles d'être utilisés pour la mise en oeuvre du procédé de l'invention.

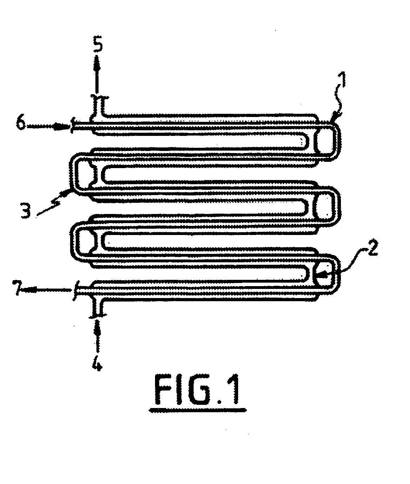

La figure 1 représente un réacteur tubulaire formé de tubes concentriques.

Ainsi, le réacteur est constitué d'un tube (1) à l'intérieur duquel circulent les réactifs en mélange qui entrent en (6) et les produits qui sortent en (7).

L'échange thermique est assuré par un fluide caloporteur, circulant dans une double enveloppe (2), qui entre en (4) et qui sort en (5). Il s'agit généralement de l'eau ou un solvant organique approprié comme par exemple un éther aromatique comme l'oxyde de biphényle et/ou l'oxyde de benzyle, une huile paraffinique et/ou naphténique, résidus de distillation du pétrole, etc...

Le tube peut contenir des tronçons de garnissage après chaque coude (3).

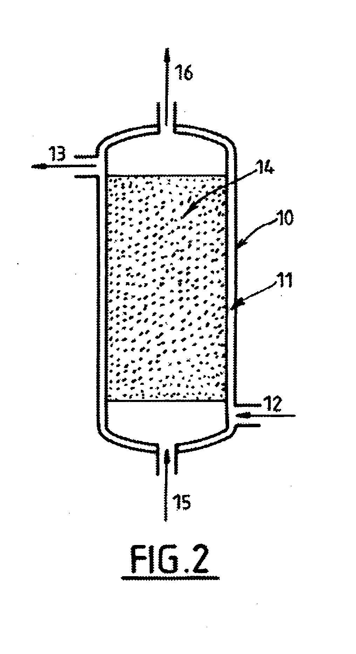

La figure 2 représente un réacteur en forme de colonne (10) équipé d'une double enveloppe (11) ou tout moyen équivalent, dans laquelle circule un fluide caloporteur qui entre en (12) et sort en (13).

La colonne est munie d'un garnissage (14).

Les réactifs sont introduits en (15) et les produits ressortent en (16).

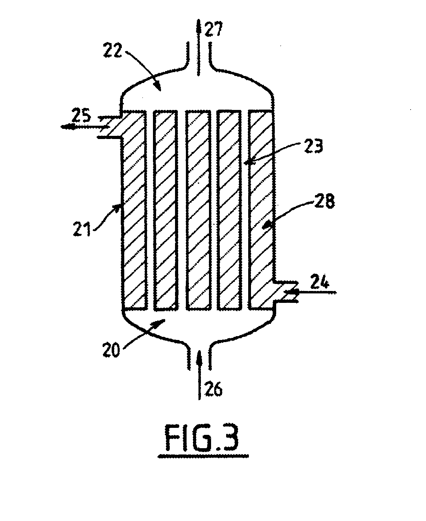

Il est à noter que l'invention n'exclut pas le cas où le réacteur tubulaire représenté par la figure 3 soit du type échangeur à calandre multitubulaire vertical.

Il comprend une zone d'introduction des réactifs (20), une zone centrale (21) et une zone de sortie des réactifs (22). La zone centrale comprend un faisceau de tubes parallèles (23) ; chaque tube comportant une entrée débouchant dans la zone d'entrée et une sortie débouchant dans la zone de sortie.

Le fluide caloporteur entrant en (24) et sortant en (25) circule dans la calandre (28) autour des tubes.

Les réactifs sont introduits en (26) et les produits ressortent en (27).

Les tubes peuvent ou non être équipés de garnissage.

Les appareillages proposés sont particulièrement utiles pour le procédé décrit car ils permettent de maintenir un écoulement piston pour une réaction qui nécessite un long temps de séjour, avec des vitesses d'écoulement faibles.

Par ailleurs, il permet de limiter les réactions subséquentes conduisant à des composés secondaires non désirés L'invention sera expliquée plus en détail au moyen de l'exemple ci-après d'un mode préféré de réalisation de l'invention, donné à titre non limitatif.

EXEMPLES

Sauf indication contraire, les pourcentages indiqués sont exprimés en poids.

Dans les exemples, on définit le taux de conversion, le rendement et la sélectivité obtenus.

Le taux de conversion (TT) correspond au rapport entre le nombre de réactif (acide glyoxylique) transformées et le nombre de moles de réactif (acide glyoxylique) engagées.

La sélectivité (RT) correspond au rapport entre le nombre de moles de produit formées (acide 4-hydroxy-3-méthoxy-mandélique) et le nombre de moles de réactif (acide glyoxylique) transformées.

Exemple 1

Dans un réacteur de 6 litres muni d'une double-enveloppe, d'une électrode de pH, d'une sonde de température, d'un réfrigérant, d'une arrivée de gaz inerte et d'une agitation mécanique, on alimente :

- 41 kg/h d'eau distillée,
- 8,9 kg/h d'une solution aqueuse de soude à 30 % en poids,
- 5,48 kg/h de gaïacol
- 3,85 kg/h d'une solution aqueuse d'acide glyoxylique à 50 % en poids.

Cette solution est ensuite alimentée avec un débit de 58l/h dans un réacteur tubulaire tel qu'illustré par la figure 2, d'un volume d'environ 15 litres (longueur de 2 m et diamètre de 100 mm), garni d'un garnissage Sulzer SMX.

La température du réacteur piston est maintenue à 38°C.

Dans ces conditions, le temps de séjour dans le réacteur est de 16 minutes et le nombre de Reynolds est de 140.

A la sortie du réacteur tubulaire, on dose les produits de la réaction par chromatographie liquide haute performance.

Les résultats obtenus sont consignés dans le tableau ci-dessous.

On n'observe pas d'encrassement du réacteur, même au bout de plusieurs mois.

Exemple 2 (exemple comparatif)

On reproduit l'exemple 1 à la différence que l'on n'introduit pas le mélange réactionnel du premier réacteur dans un réacteur tubulaire mais dans une cascade de deux réacteurs de type parfaitement agités équipés d'un agitateur à hélice à 4 pales inclinées, chaque réacteur ayant un volume de 10 litres.

A la sortie du dernier réacteur, on dose les produits de la réaction par chromatographie liquide haute performance. Les résultats obtenus sont consignés dans le tableau ci-dessous.

Tableau (I)

| Réf. Ex. | Conversion | Acide 4-hydroxy-3-méthoxy-mandélique | Acide 2-hydroxy-3-méthoxy-mandélique | Acide 2-hydroxy-3-méthoxy-1,5-dimandélique |
|---|---|---|---|---|
| 1 | TT = 72,2 % | RT=81,9% | RT=6,1 % | RT = 11,1 %. |
| 2 | TT = 71,9% | RT = 79,3 % | RT = 5,8% | RT = 14,7% |

On constate que pour un taux de conversion équivalent, la sélectivité en produit recherché, l'acide 4-hydroxy-3-méthoxymandélique, estplus élevée pour un réacteurtubulaire que pour une cascade de réacteurs de type parfaitement agités.

L'utilisation d'un réacteur tubulaire comportant un garnissage permet donc d'augmenter la sélectivité du procédé de préparation de composés p-hydroxymandéliques comme l'acide 4-hydroxy-3-méthoxymandélique.

**Revendications**

1. Procédé de préparation de vanilline par :

    - préparation de l'acide 4-hydroxy-3-méthoxymandélique, comprenant la condensation dans l'eau, en présence d'un agent alcalin, de gaïacol, avec l'acide glyoxylique, **caractérisé par le fait que** la réaction est conduite dans un réacteur à écoulement piston,
    - et oxydation de l'acide 4-hydroxy-3-méthoxymandélique obtenu.

2. Procédé de préparation d'éthylvanilline par :

    - préparation de l'acide 3-éthoxy-4-hydroxymandélique, comprenant la condensation dans l'eau, en présence d'un agent alcalin, de guétol, avec l'acide glyoxylique, **caractérisé par le fait que** la réaction est conduite dans un réacteur à écoulement piston,
    - et oxydation de l'acide 3-éthoxy-4-hydroxymandélique obtenu.

3. Procédé selon la revendication 1 ou la revendication 2, **caractérisé par le fait que** le réacteur ne comporte pas de garnissage lorsque l'écoulement dans le réacteur présente un nombre de Reynolds supérieur ou égal à 2000 et de préférence supérieur à 5000.

4. Procédé selon la revendication 1 ou la revendication 2, **caractérisé par le fait que** le réacteur comporte un garnissage lorsque l'écoulement dans le réacteur présente un nombre de Reynolds inférieur à 2000.

5. Procédé selon l'une des revendications 3 et 4, **caractérisé par le fait que** le réacteur présente un rapport longueur / diamètre compris entre 4 et 30.

6. Procédé selon la revendication 5, **caractérisé par le fait que** le réacteur présente un rapport longueur / diamètre compris entre 5 et 10.

7. Procédé selon l'une des revendications 1 à 6, **caractérisé par le fait que** le réacteur est un réacteur tubulaire formé de tubes concentriques, un réacteur en forme de colonne ou un réacteur tubulaire comprenant des tubes réunis en faisceau.

8. Procédé selon la revendication 7, **caractérisé par le fait que** le réacteur est un réacteur colonne.

9. Procédé selon la revendication 4, **caractérisé par le fait que** le garnissage est un garnissage de type mélangeur statique.

10. Procédé selon la revendication 4, **caractérisé par le fait que** le garnissage est composé d'éléments de mélange comportant des lames de guidage arrangés à des angles précis et arrangés de manière complexe et commercialisé par la société SULZER sous la dénomination SMV et SMX.

**11.** Procédé selon l'une des revendications 1 à 10 **caractérisé par le fait que** le rapport molaire entre le gaïacol ou le guétol et l'acide glyoxylique varie entre 1,0 et 4,0.

**12.** Procédé selon la revendication 11 **caractérisé par le fait que** la viscosité du mélange à 20°C est comprise entre 1,5 et 3 mPa.

**13.** Procédé selon l'une des revendications 1 à 12 **caractérisé par le fait que** la température de la réaction varie entre 20°C et 90°C, de préférence entre 30°C et 40°C.

## Patentansprüche

**1.** Verfahren zur Herstellung von Vanillin durch :

- Herstellung von 4-Hydroxy-3-methoxymandelsäure, umfassend die Kondensation von Guajacol mit Glyoxylsäure in Wasser in Gegenwart eines alkalischen Mittels, **dadurch gekennzeichnet, dass** die Reaktion in einem Pfropfenströmungsreaktor durchgeführt wird,
- und Oxididation der erhaltenen 4-Hydroxy-3-methoxymandelsäure.

**2.** Verfahren zur Herstellung von Ethylvanillin durch :

- Herstellung von 3-Methoxy-4-hydroxy-mandelsäure, umfassend die Kondensation von Guetol mit Glyoxylsäure in Wasser in Gegenwart eines alkalischen Mittels, **dadurch gekennzeichnet, dass** die Reaktion in einem Pfropfenströmungsreaktor durchgeführt wird,
- und Oxidation der erhaltenen 3-Methoxy-4-hydroxy-mandelsäure.

**3.** Verfahren nach Anspruch 1 oder Anspruch 2, **dadurch gekennzeichnet, dass** der Reaktor keine Packung umfasst, wenn die Strömung in dem Reaktor eine Reynolds-Zahl von größer oder gleich 2000 und vorzugsweise mehr als 5000 aufweist.

**4.** Verfahren nach Anspruch 1 oder Anspruch 2, **dadurch gekennzeichnet, dass** der Reaktor eine Packung umfasst, wenn die Strömung in dem Reaktor eine Reynolds-Zahl von weniger als 2000 aufweist.

**5.** Verfahren nach einem der Ansprüche 3 und 4, **dadurch gekennzeichnet, dass** der Reaktor ein Länge/Durchmesser-Verhältnis zwischen 4 und 30 aufweist.

**6.** Verfahren nach Anspruch 5, **dadurch gekennzeichnet, dass** der Reaktor ein Länge/Durchmesser-Verhältnis zwischen 5 und 10 aufweist.

**7.** Verfahren nach einem der Ansprüche 1 bis 6, **dadurch gekennzeichnet, dass** es sich bei dem Reaktor um einen Rohrreaktor aus konzentrischen Rohren, einen Reaktor in Säulenform oder einen Rohrreaktor mit gebündelten Rohren handelt.

**8.** Verfahren nach Anspruch 7, **dadurch gekennzeichnet, dass** es sich bei dem Reaktor um einen Säulenreaktor handelt.

**9.** Verfahren nach Anspruch 4, **dadurch gekennzeichnet, dass** es sich bei der Packung um eine Packung vom Typ statischer Mischer handelt.

**10.** Verfahren nach Anspruch 4, **dadurch gekennzeichnet, dass** die Packung aus Mischelementen besteht, die auf genaue Winkel eingestellte Leitflügel umfassen und komplex angeordnet sind und von der Firma SULZER unter der Bezeichnung SMV und SMX vertrieben werden.

**11.** Verfahren nach einem der Ansprüche 1 bis 10, **dadurch gekennzeichnet, dass** das Molverhältnis zwischen Guajacol oder Guetol und der Glyoxylsäure zwischen 1,0 und 4,0 liegt.

**12.** Verfahren nach Anspruch 11, **dadurch gekennzeichnet, dass** die Viskosität der Mischung bei 20°C zwischen 1,5 und 3 mPa liegt.

13. Verfahren nach einem der Ansprüche 1 bis 12, **dadurch gekennzeichnet, dass** die Reaktionstemperatur zwischen 20°C und 90°C; vorzugsweise zwischen 30°C und 40°C, liegt.

**Claims**

1. Process for preparing vanillin by :

   - preparing 4-hydroxy-3-methoxymandelic acid, comprising the condensation in water , in the presence of an alkaline agent, of gaiacol, with glyoxylic acid, **characterized in that** the reaction is performed in a piston-flow reactor,
   - and oxidizing the obtained 4-hydroxy-3-methoxymandelic acid.

2. Process for preparing ethylvanillin by :

   - preparing 3-ethoxy-4-hydroxymandelic acid, comprising the condensation in water , in the presence of an alkaline agent, of guetol, with glyoxylic acid, **characterized in that** the reaction is performed in a piston-flow reactor,
   - and oxidizing the obtained 3-ethoxy-4-hydroxymandelic acid.

3. Process according to Claim 1 or Claim 2, **characterized in that** the reactor does not comprise any packing when the flow in the reactor has a Reynolds number greater than or equal to 2000 and preferably greater than 5000.

4. Process according to Claim 1 or Claim 2, **characterized in that** the reactor comprises packing when the flow in the reactor has a Reynolds number of less than 2000.

5. Process according to either of Claims 3 and 4, **characterized in that** the reactor has a length/diameter ratio of between 4 and 30.

6. Process according to Claim 5, **characterized in that** the reactor has a length/diameter ratio of between 5 and 10.

7. Process according to one of Claims 1 to 6, **characterized in that** the reactor is a tubular reactor formed from concentric tubes, a reactor of column shape or a tubular reactor comprising tubes bundled together in an array.

8. Process according to Claim 7, **characterized in that** the reactor is a column reactor.

9. Process according to Claim 4, **characterized in that** the packing is packing of static mixer type.

10. Process according to Claim 4, **characterized in that** the packing is composed of mixing elements comprising guide blades set at precise angles and arranged in a complex manner, and sold by the company Sulzer under the names SMV and SMX.

11. Process according to one of Claims 1 to 10, **characterized in that** the mole ratio between the gaiacol or the guétol and the glyoxylic acid ranges between 1.0 and 4.0.

12. Process according to Claim 11, **characterized in that** the viscosity of the mixture at 20°C is between 1.5 and 3 mPa.

13. Process according to one of Claims 1 to 12, **characterized in that** the reaction temperature ranges between 20°C and 90°C and preferably between 30°C and 40°C.

# FIG.1

FIG.2

FIG.3

**EP 2 222 627 B3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9965853 A
- FR 2379501 A
- JP 51128934 A, NAGAI SHIGEKI

**Littérature non-brevet citée dans la description**

- KIRK-OTHMER - Encyclopedia of Chemical Technology. 1997, vol. 24, 812-825
- **M. SMITH ; J. MARCH.** Advanced Organic Chemistry. John Wiley and Sons, 1992, 46
- **P. HEBERT.** *Bull. Soc. Chim. France,* 1920, vol. 27, 45-55
- Don't Be Baffled By Static Mixers. *Chemical Engineering,* Mai 2003